# EUROPEAN PATENT APPLICATION

(11) **EP 2 957 308 A1**
(43) Date of publication of application: **23.12.2015**
(21) Application number: 14172867.5
(22) Date of filing: 18.06.2014
(51) Int. Cl.: A61M 5/24

(54) **Cartridge holder for a drug delivery device**

(71) Applicant: Sanofi-Aventis Deutschland GmbH, 65929 Frankfurt am Main (DE)
(72) Inventor: Jugl, Michael, 65926 Frankfurt am Main (DE); Teucher, Axel, 65926 Frankfurt am Main (DE)

(57) **Abstract**

The present invention relates to a cartridge holder of a drug delivery device, comprising:
- a cartridge holder body (14) extending in a longitudinal axial direction (1, 2) to accommodate a cartridge (18) filled with a medicament,
- an ejector (32) displaceably arranged inside the cartridge holder body (14) between a distal stop position (3) and a proximal stop position (4) and being operably engageable with the cartridge (18) to displace the cartridge (18) in proximal direction (2) relative to the cartridge holder body (14).

## Description

### Field of the invention

The present invention relates to the field of cartridge holders for drug delivery devices and in particular to injection devices equipped with a replaceable cartridge containing a medicament to be delivered by way of injection. The invention further relates to a drug delivery device, in particular to a reusable injection device, such like a pen-type injector

### Background

Injection devices for setting and dispensing a single or multiple doses of a liquid medicament are as such well-known in the art. Generally, such devices have substantially a similar purpose as that of an ordinary syringe.

Injection devices, in particular pen-type injectors have to meet a number of user-specific requirements. For instance, with patient's suffering chronic diseases, such like diabetes, the patient may be physically infirm and may also have impaired vision. Suitable injection devices especially intended for home medication therefore need to be robust in construction and should be easy to use. Furthermore, manipulation and general handling of such a device and its components should be intelligible and easy understandable. Moreover, a dose setting as well as a dose dispensing procedure must be easy to operate and has to be unambiguous.

Typically, such devices comprise a housing and/or a particular cartridge holder, adapted to receive a cartridge at least partially filled with the medicament to be dispensed. The device further comprises a drive mechanism, usually having a displaceable piston rod, which is adapted to operably engage with a piston of the cartridge. By means of the drive mechanism and its piston rod, the piston of the cartridge is displaceable in a distal or dispensing direction and may therefore expel a predefined amount of the medicament via a piercing assembly, which is to be releaseably coupled with a distal end section of the housing, typically of the cartridge holder of the injection device.

The medicament to be dispensed by the injection device is provided and contained in a cartridge. Such cartridges typically comprise a vitreous barrel sealed in distal direction by means of a pierceable seal and being further sealed in proximal direction by the piston. With reusable injection devices an empty cartridge is replaceable by a new one. In contrast to that, injection devices of disposable type are to be entirely discarded when the medicament in the cartridge has been dispensed or used-up.

With such reusable drug delivery devices or injection devices, such like pen-type injectors an empty cartridge has to be frequently replaced. The cartridge typically located and accommodated in a cartridge holder forming a distal portion of the housing of such a drug delivery device is to be disconnected from a proximal housing component of the device. Since the cartridge is completely contained inside the disconnected cartridge holder, it is not directly accessible from outside the cartridge holder. In particular in situations where the empty cartridge is subject to tilt or cant and where the cartridge may be clamped inside the cartridge holder, its removal or withdrawal out of the disconnected cartridge holder might be rather sophisticated and cumbersome. In such situations an end user of the device, hence a patient may be encouraged to tap out the cartridge from the cartridge holder, e.g. by beating the inverted cartridge holder on e.g. a table or other rigid structure.

Such a tapping out may not only harm the integrity of the cartridge, which typically comprises a vitreous barrel but may also lead to damages of the cartridge holder and hence of the entire drug delivery device. It is therefore an object of the present invention to provide an improved cartridge holder which allows for an easy, intuitive and product safe removal of the cartridge therefrom. It is a further object to provide a respective drug delivery device. The improvement to the cartridge holder should be rather simple, lightweight, cost efficient, reliable and long term stable.

### Summary of the invention

In a first aspect the invention relates to a cartridge holder of a drug delivery device, typically to a cartridge holder of an injection device, such like a pen-type injector. The cartridge holder comprises a cartridge holder body extending in a longitudinal axial direction to accommodate a cartridge filled with the medicament. Generally, the cartridge holder may be of arbitrary design and shape. Typically, it comprises a cross-section and overall design that generally matches with the geometry and overall design of the cartridge to be assembled therein. For a cartridge of tubular design the cartridge holder typically comprises a tubular or cylindrical structure with its cylinder long axis defining the longitudinal axial direction. The cartridge is then to be assembled in an orientation wherein the longitudinal or axial direction of the cartridge co-aligns with the axial or longitudinal direction of the cartridge holder.

The cartridge holder further comprises an ejector displaceably arranged inside the cartridge holder body between a distal stop position and a proximal stop position. The ejector is operably engageable with the cartridge to displace the cartridge in proximal direction relative to the cartridge holder body. The ejector which is directly and mechanically engageable with the cartridge allows to apply a proximally directed driving force to the cartridge in order to remove the cartridge from a proximal end of the cartridge holder, hence of its cartridge holder body. By means of the ejector and in particular by means of a well-defined handling and displacement of the ejector from its distal stop position towards and into its proximal stop position also the cartridge directly engaged with the ejector is displaced in proximal direction so that a proximal end thereof protrudes from a proximal end of the cartridge holder. With the proximal end of the cartridge protruding from the cartridge holder, the cartridge can be easily gripped and taken out of the cartridge holder for replacement by a new cartridge.

There are various embodiments conceivable for the ejector. For instance, the ejector may comprise a flexible strap which is seizable or accessible by a user as soon as the cartridge holder, hence its cartridge holder body is disconnected and detached from a proximal housing component of the drug delivery device. Tearing of such a strap may be already sufficient to induce a displacement of the cartridge in proximal direction relative to the cartridge holder and hence relative to the cartridge holder body.

According to another embodiment the ejector comprises an ejector body that is slidably arranged inside the cartridge holder body in axial direction. Typically, the ejector body is displaceable between the distal stop position and the proximal stop position of the ejector. Moreover, the rather rigid ejector body actually forms and constitutes the ejector.

By having the ejector body slidably arranged inside the cartridge holder in proximal and distal direction, hence in opposite axial directions, a sliding displacement of the cartridge relative to the cartridge holder body is inducible by the ejector.

The ejector is displaceable between the distal and proximal stop position relative to the cartridge holder body to such an extent, that the proximal end of the cartridge is completely located inside the cartridge holder body when the ejector is in its distal stop position. When the ejector arrives at the proximal stop position the proximal end of the cartridge typically protrudes to such an extent from the proximal end of the cartridge holder that the cartridge is grippable and sufficiently seizable by a person, hence by the patient. It is then, that the cartridge can be safely seized and gripped by the end user so as to safely withdraw the cartridge from the cartridge holder.

According to a further embodiment the ejector comprises an abutment to axially abut with a neck portion of the cartridge. Typically, a vitreous cartridge has a tubular barrel, which towards its distal end radially narrows and forms a kind of a neck portion comparable to a bottle neck. The ejector, typically arranged near and at least partially surrounding a distal end of the cartridge comprises an inwardly, typically a radially inwardly extending abutment to axially engage with the narrowing neck portion of the cartridge. The abutment as well as the cartridge's neck portion at least in sections extend in radial direction, i.e. in a plane perpendicular to the longitudinal axial direction so that an axial and user-induced displacement of the ejector relative to the cartridge holder body is unalterably and directly transferable to a respective relative displacement of cartridge and cartridge holder body.

The abutment of the ejector is particularly adapted to the shape and geometry of the cartridge, in particular of its neck portion. In this way, a particularly well-defined ejection displacement of the cartridge can be obtained. It is generally conceivable, that the abutment comprises various abutment portions or abutment elements being regularly or irregularly arranged around or along the outer circumference of the cartridge and/or of its distal neck portion. In this way, any proximally acting displacement forces transferred from the ejector to the cartridge can be homogenously distributed across the cartridge's circumference. It is furthermore conceivable, that the abutment comprises an annular shape completely surrounding the cartridge's neck portion.

According to a further embodiment the abutment comprises a radially inwardly extending flange portion that is located at a distal end of the ejector, hence at a distal end of the ejector body. The radially inwardly extending flange portion may comprise various and circumferentially or tangentially separated flange elements, by way of which a multiple abutment between the abutment and the cartridge is obtainable. It is also conceivable, that the flange portion is of annular shape and completely surrounds the outer circumference of the cartridge. Typically, the flange portion at the distal end of the ejector extends in a plane perpendicular to the longitudinal axial direction so that a mutual axial abutment between cartridge and ejector is attainable.

According to another embodiment the ejector comprises a through opening to receive a radially narrowing neck portion of the cartridge. The through opening is typically provided in a distal end face of the ejector. The through opening may correspond to and may be formed by the above mentioned radially inwardly extending flange portion of the ejector, hence of the ejector body. It is particularly conceivable, that the ejector comprises a sleeve-shaped insert slidably arranged inside a distal portion of the cartridge holder. A distal end face of such an ejector is then typically provided with a central through opening through which the radially narrowing neck portion of the cartridge may reach through to a certain extent until shoulder portions of the neck portion get in axial abutment with proximally facing portions of the radially inwardly extending flange portion or of a portion of the ejector surrounding the through opening.

By means of the through opening, also an improved fitting and fastening of the cartridge inside the cartridge holder can be obtained. It is even conceivable, that distally directed dispensing forces acting on a piston of the cartridge during dispensing of a dose are transferred via the ejector to the cartridge holder. In this way it can be effectively prevented, that e.g. a very distal end of the cartridge axially abuts with an inward facing portion of the distal end section of the cartridge holder. Hence, through the cartridge's abutment with the ejector in the region of the radially narrowing neck portion mechanical loads otherwise acting on the sealed distal end of the cartridge can be minimized.

According to another embodiment the ejector comprises a slider seizable from outside the cartridge holder body. By way of the slider, the ejector gets accessible to the end user. An end user may directly interact with the slider, which is seizable and accessible from outside the cartridge holder, hence from outside the cartridge holder body. Access to the slider may be provided in various different ways. By means of a user directly interacting with the slider, the slider and hence the ejector operably and directly connected therewith is transferable from its distal stop position towards and into its proximal stop position, thereby inducing a proximally directed sliding motion of the cartridge relative to the cartridge holder.

According to another embodiment the slider comprises a radially outwardly extending handle portion. Said handle portion is located in or extends through a longitudinally extending through opening or it extends through a longitudinally extending slit of a sidewall of the cartridge holder body. The sidewall of the cartridge holder body either comprises a longitudinally extending through opening or a respective longitudinally extending slit of sufficient size in order to directly provide access to the handle portion located inside or underneath the through opening. It is generally conceivable and of further advantage for an easy and intuitive handling of the ejector when the handle portion of the slider and hence of the ejector radially outwardly protrudes from the through opening or from a respective longitudinally extending slit.

In this way, an even more easy access from outside the cartridge holder can be provided. A radially outwardly extending handle portion is of particular benefit to induce a proximally directed ejection force to the ejector, to the ejector body and hence to the cartridge which is operably and mechanically engaged therewith.

In particular with a comparatively small slit in the cartridge holder body's sidewall ingress of contaminating particles or humidity into the cartridge holder can be reduced to a minimum. It is furthermore conceivable, that the slider's handle portion is larger in size than the width of the slit or of the through opening. In this way, the through opening or slit in the sidewall of the cartridge holder body can be effectively covered or it can be effectively sealed by a correspondingly-shaped handle portion. In this way, the through opening or slit in the sidewall of the cartridge holder body can be covered, thereby effectively hiding the ejector mechanism and simultaneously providing an attractive design of the cartridge holder and hence of the drug delivery device.

According to another embodiment the ejector's proximally directed displacement is delimited by means of the slider or its handle portion that axially abut with a proximal end of the through opening or slit of the cartridge holder body. In this way, the through opening or slit provides a twofold function. First of all, it allows to provide access to the ejector and hence to the ejector body from outside the cartridge holder and hence from outside the drug delivery device. Second, the longitudinal extension and geometry of the through opening or of a respective slit directly defines the maximum distance as well as the absolute or relative positions of distal and proximal stop position of the ejector relative to the cartridge holder body.

In another embodiment, the slider is integrally formed with the ejector body. Typically, the slider is provided and designed as a radially outwardly extending flange portion or protrusion of the sleeve-shaped ejector body. By having the slider integrally formed with the ejector body and further by having the inwardly extending flange portion integrally formed with the ejector body, any forces or axial displacement acting on the slider can be directly and unalterably transferred to the cartridge itself.

According to another embodiment the slider and hence the handle portion thereof is or are located proximally offset from a distal end of the ejector. Typically, the slider is located at a proximal offset from the abutment or from the flange portion of the ejector. The slider may be particularly provided at or near a proximal end of the ejector. Typically, the longitudinal extension of the ejector or of the ejector body is larger than the longitudinal extension of the sidewall's through opening or slit. In this way and during displacement of the ejector from its distal stop position towards its proximal stop position the ejector body effectively covers the sidewall's through opening or slit. The integral, hence unitary design of slider and ejector body is also beneficial in terms of manufacturing of the cartridge holder. Ejector body and slider together with the handle portion can be provided as a single piece, which is to be assembled inside the cartridge holder during a manufacturing process.

Typically, the ejector of sleeve-like design comprises an outer diameter that matches or which is slightly smaller than the inner free diameter of the cartridge holder body. In this way, a cant-free and tumble-free as well as a rather smooth running sliding arrangement of the ejector inside the cartridge holder body can be obtained. Typically, the cartridge holder as well as the ejector is made and manufactured as an injection molded plastic component. Such an implementation is rather beneficial for a cost-efficient mass manufacturing process.

According to another embodiment the slider is located proximally offset from a distal end of the ejector. This allows to provide a through opening or longitudinal slit for the slider at a location in or on the sidewall of the cartridge holder body at a certain distance from the cartridge holder's distal end. Typically, the distal end of the cartridge holder comprises a well-defined and standardized connecting structure to releasably and detachably connect a needle assembly to the cartridge holder. With the proximally located offset of the slider from the distal end of the ejector such standardized and well-defined interconnecting structures can be left unmodified. Constructional and design modifications for implementing the ejector can take place at almost any arbitrary position of the cartridge holder body.

According to a further embodiment the cartridge holder also comprises a cartridge filled with a medicament, wherein said cartridge is assembled inside the cartridge holder. The cartridge, which is typically of vitreous type and which typically comprises a tubular barrel may even frictionally engage with an inside facing sidewall portion of the ejector. In this way, a rather slip-free and precise mechanical interaction between the ejector and the cartridge can be obtained. Moreover, by means of a friction coupling and tightly fitting engagement of ejector and cartridge also the position of the ejector inside the cartridge holder can be fixed. Even in the event that the ejector would be rather loosely fitted inside the cartridge holder, a rather immobile and fixed arrangement of the ejector could be obtained via a tight fitting, e.g. frictional engagement of ejector and cartridge.

According to a further embodiment a proximal end of the cartridge proximally protrudes from a proximal end of the cartridge holder when the ejector is in its proximal stop position. Typically, the proximal end of the cartridge lies within the outer circumference or proximal end of the cartridge holder when the ejector is in its distal stop position. When located and arranged in its proximal stop position the proximal end of the cartridge proximally and hence axially protrudes from a respective proximal end of the cartridge holder. It is therefore easily and directly seizable and grippable, so that a product safe withdrawal and removal of the cartridge from the cartridge holder can be provided. Displacement of the ejector from its proximal stop position towards and into its distal stop position is attainable via a displacement of the cartridge into distal direction and through the mutual axial abutment of cartridge and ejector.

According to a further aspect the invention also relates to a drug delivery device and in particular to an injection device, e.g. of pen-injector type. The drug delivery device comprises a proximal housing to accommodate a drive mechanism which is operably engageable with a piston of a cartridge, wherein said cartridge is at least partially filled with a medicament. The drug delivery device further comprises a cartridge holder as described above and which is operable and designed to accommodate the cartridge. The cartridge holder further comprises a proximal end to releasably connect with a distal end of the proximal housing of the drug delivery device. Typically, proximal end of the cartridge holder as well as the distal end of the proximal housing of the drug delivery device geometrically mutually correspond and comprise mutually corresponding mating structures that allow for a releasable engagement of proximal housing and cartridge holder as well as for a detachable mutual connection thereof.

The drug delivery device is typically of reusable type. Proximal housing and cartridge holder are detachably interconnectable so as to enable replacement of an empty cartridge.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 Exendin-4(1-39),
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(O2)25, Asp28] Exendin-4(1-39),
   des Pro36 [Trp(O2)25, IsoAsp28] Exendin-4(1-39),
   des Pro36 [Met(O)14 Trp(O2)25, Asp28] Exendin-4(1-39),
   des Pro36 [Met(O)14 Trp(O2)25, IsoAsp28] Exendin-4(1-39),
   wherein the group -Lys6-NH2 may be bound to the C-terminus of the Exendin-4 derivative;
or an Exendin-4 derivative of the sequence
   des Pro36 Exendin-4(1-39)-Lys6-NH2 (AVE0010),
   H-(Lys)6-des Pro36 [Asp28] Exendin-4(1-39)-Lys6-NH2,
   des Asp28 Pro36, Pro37, Pro38Exendin-4(1-39)-NH2,
   H-(Lys)6-des Pro36, Pro38 [Asp28] Exendin-4(1-39)-NH2,
   H-Asn-(Glu)5des Pro36, Pro37, Pro38 [Asp28] Exendin-4(1-39)-NH2,
   des Pro36, Pro37, Pro38 [Asp28] Exendin-4(1-39)-(Lys)6-NH2,
   H-(Lys)6-des Pro36, Pro37, Pro38 [Asp28] Exendin-4(1-39)-(Lys)6-NH2,
   H-Asn-(Glu)5-des Pro36, Pro37, Pro38 [Asp28] Exendin-4(1-39)-(Lys)6-NH2,
   H-(Lys)6-des Pro36 [Trp(O2)25, Asp28] Exendin-4(1-39)-Lys6-NH2,
   H-des Asp28 Pro36, Pro37, Pro38 [Trp(O2)25] Exendin-4(1-39)-NH2,
   H-(Lys)6-des Pro36, Pro37, Pro38 [Trp(O2)25, Asp28] Exendin-4(1-39)-NH2,
   H-Asn-(Glu)5-des Pro36, Pro37, Pro38 [Trp(O2)25, Asp28] Exendin-4(1-39)-NH2,
   des Pro36, Pro37, Pro38 [Trp(O2)25, Asp28] Exendin-4(1-39)-(Lys)6-NH2,
   H-(Lys)6-des Pro36, Pro37, Pro38 [Trp(O2)25, Asp28] Exendin-4(1-39)-(Lys)6-NH2,
   H-Asn-(Glu)5-des Pro36, Pro37, Pro38 [Trp(O2)25, Asp28] Exendin-4(1-39)-(Lys)6-NH2,
   H-(Lys)6-des Pro36 [Met(O)14, Asp28] Exendin-4(1-39)-Lys6-NH2,
   des Met(O)14 Asp28 Pro36, Pro37, Pro38 Exendin-4(1-39)-NH2,
   H-(Lys)6-desPro36, Pro37, Pro38 [Met(O)14, Asp28] Exendin-4(1-39)-NH2,
   H-Asn-(Glu)5-des Pro36, Pro37, Pro38 [Met(O)14, Asp28] Exendin-4(1-39)-NH2,
   des Pro36, Pro37, Pro38 [Met(O)14, Asp28] Exendin-4(1-39)-(Lys)6-NH2,
   H-(Lys)6-des Pro36, Pro37, Pro38 [Met(O)14, Asp28] Exendin-4(1-39)-(Lys)6-NH2,
   H-Asn-(Glu)5 des Pro36, Pro37, Pro38 [Met(O)14, Asp28] Exendin-4(1-39)-(Lys)6-NH2,
   H-Lys6-des Pro36 [Met(O)14, Trp(O2)25, Asp28] Exendin-4(1-39)-Lys6-NH2,
   H-des Asp28 Pro36, Pro37, Pro38 [Met(O)14, Trp(O2)25] Exendin-4(1-39)-NH2,
   H-(Lys)6-des Pro36, Pro37, Pro38 [Met(O)14, Asp28] Exendin-4(1-39)-NH2,
   H-Asn-(Glu)5-des Pro36, Pro37, Pro38 [Met(O)14, Trp(O2)25, Asp28] Exendin-4(1-39)-NH2,
   des Pro36, Pro37, Pro38 [Met(O)14, Trp(O2)25, Asp28] Exendin-4(1-39)-(Lys)6-NH2,
   H-(Lys)6-des Pro36, Pro37, Pro38 [Met(O)14, Trp(O2)25, Asp28] Exendin-4(S1-39)-(Lys)6-NH2,
   H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (~150kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

It will be further apparent to those skilled in the art that various modifications and variations can be made to the present invention without departing from the spirit and scope of the invention. Further, it is to be noted, that any reference numerals used in the appended claims are not to be construed as limiting the scope of the invention.

### Brief description of the drawings

In the following, an exemplary embodiment of the present invention is described in detail by making reference to the Figures in which:
- Fig. 1: schematically illustrates an exploded view of the drug delivery device,
- Fig. 2: shows a cross-section through the cartridge holder with the ejector in its distal stop position and
- Fig. 3: shows a cross-section according to Fig. 2 with the ejector in its proximal stop position.

### Detailed description

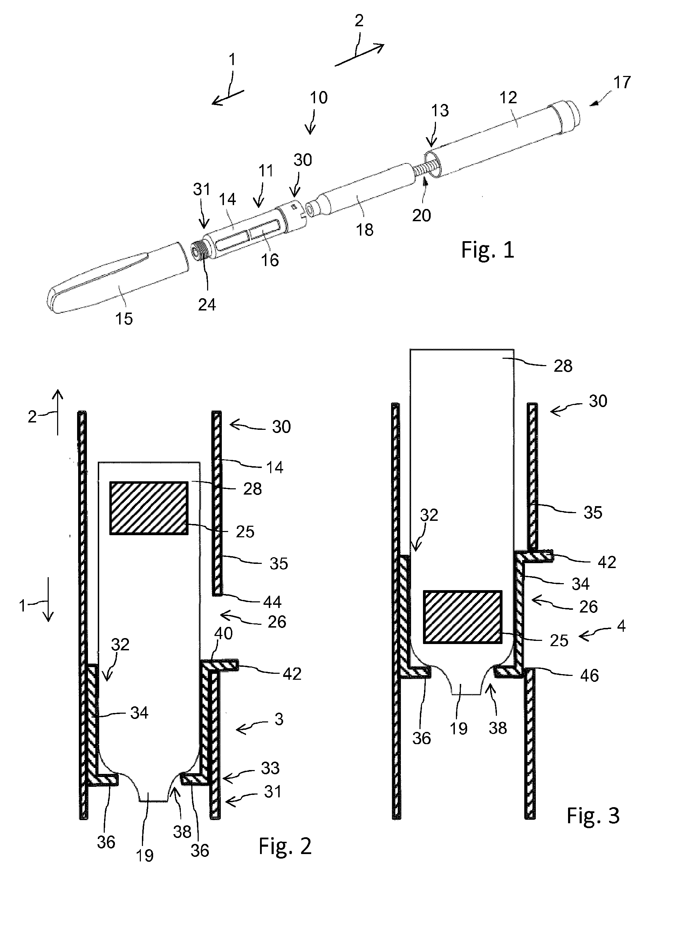

The drug delivery device 10 as illustrated in exploded view in Fig. 1 comprises a proximal housing 12 and a cartridge holder 11 detachably interconnectable therewith. While the proximal housing 12 accommodates a drive mechanism 20 having inter alia a longitudinally extending piston rod 22 the cartridge holder 11 is designed to accommodate a cartridge 18 which is at least partially filled with a medicament. The tubular body 14 of the cartridge holder 11, hence the cartridge holder body 14 comprises an inspection window 16 in order to visually inspect the cartridge 18 located therein. The drug delivery device 10 is presently shown in form of an injection device and in particular as a pen-type injector. However, the concept of the present invention is generally transferable also to other drug delivery devices in which a cartridge 18 is replaceably arranged.

The very distal end of the drug delivery device 10 is covered with a removable protective cap 15. In general, the distal longitudinal direction is denoted with reference 1 while the proximal direction is indicated with reference number 2. The distal direction points towards the patient and towards biological tissue, into which a dose of the medicament may be injected with the aid of the drug delivery device 10. The distal end 31 of the cartridge holder 11 comprises a threaded socket 24 in order to detachably connect to a needle assembly, which is not particularly illustrated here. The needle assembly typically comprises a double-tipped needle, wherein a proximal end of the needle intersects a distal seal of the cartridge 18 thereby gaining access to the interior of the cartridge 18. An opposite and distal end of the double-tipped needle is then intended to puncture biological tissue and to deliver a dose of the medicament in the tissue.

The drive mechanism 20 of the drug delivery device 10 may be manually operated, electrically operated and/or may comprise some kind of energy storage means, such like a mechanical spring providing at least a contribution to a dispensing force or torque. At a proximal end 17 the proximal housing 12 is equipped with a dose dial and/or with a dose button. Operation of these functional elements may serve to set a dose of variable size and to subsequently dispense the dose via injection.

The distal end 13 of the proximal housing 12 is releasably interconnectable with the proximal end 30 of the cartridge holder 11. There are different types of interconnecting mechanisms within the scope of the present invention. Among a large variety of connecting mechanisms a threaded or snap-fit engagement of cartridge holder 11 and proximal housing 12 is conceivable.

As it is schematically illustrated in the two cross-sections according to Figs. 2 and 3 the cartridge holder 11 comprises a cartridge holder body 14, which is of substantially tubular and longitudinally elongated shape. The cartridge holder body 14 is designed to completely accommodate and to completely receive the cartridge 18. As further shown in Figs. 2 and 3, towards its proximal end 28, the cartridge 18 is sealed with a piston 25 of elastomeric material. For dispensing of a dose, the piston 25 is driven in distal direction 1 under the action of the piston rod 22 of the drive mechanism 20, thereby raising the fluid pressure inside the cartridge 18 and expelling a well-defined amount of the medicament from the cartridge 18.

The cartridge 18 comprises a neck portion 19 towards its distal end. Compared to its proximal end 28, the neck portion 19 is radially narrowed. As seen from the distal direction 1 and when starting from the distal end of the cartridge 18, the neck portion 19 forms and comprises radially widening shoulders that axially and distally abut with an inwardly, hence radially inwardly extending abutment 36 of an ejector 32. As becomes apparent from Figs. 2 and 3, the ejector 32 comprises an ejector body 34, which may be of tubular shape and which matches in size and geometry with the interior geometry of the cartridge holder body 14. The ejector 32, in particular the ejector body 34 is slidably arranged in distal and proximal direction 1, 2 inside the cartridge holder body 14. The abutment 36 provided at the distal end of the ejector body 34 forms a through opening through which the distal end, hence the neck portion 19 of the cartridge 18 at least in sections axially protrudes. By means of the e.g. annular abutment 36, the neck portion 19, hence the radially widening shoulder of the cartridge 18 may be in direct support and contact along its entire circumference with the ejector 32.

Near its proximal end, the sleeve-shaped ejector body 34 comprises a radially outwardly extending slider 40 forming or having a handle portion 42 that extends radially outwardly and reaches through a through opening 26 of a sidewall 35 of the cartridge holder body 14. The through opening 26, which may be shaped as a narrow longitudinally extending slit comprises a proximal end 44 as shown in Fig. 2 and further comprises an opposite distal end 46 as shown in Fig. 3. The radially outwardly extending handle portion 42 of the slider 40 not only allows for an easy and intuitive gripping and axial displacement of the ejector 32 relative to the cartridge holder 11 but also serves as a delimiter to block a further axial displacement of the ejector 32 when either a proximal stop position 4 as shown in Fig. 3 or when a distal stop position 3 as shown in Fig. 2 has been reached.

In the distal stop position 3 as shown in Fig. 2, which corresponds to the conventional position of the ejector 32 and the cartridge 18 during repeated dispensing actions of the drug delivery device 10, the handle portion 42 is in axial abutment with the distal end 46 of the longitudinally extending through opening 26. In this configuration, a proximal end 28 of the cartridge 18 is completely arranged inside the cartridge holder 11. The proximal end 28 is not directly accessible from outside the device 10. It requires a turn around of the cartridge holder 11, which may come along with an uncontrolled removal of the cartridge 18 from the cartridge holder 11.

In order to provide a controlled and well-defined removal and withdrawal of the cartridge 18 from the cartridge holder 11, the handle portion 42 of the ejector 32 is to be displaced in proximal direction 2 relative to the cartridge holder body 14. As it is immediately apparent from a comparison of Figs. 2 and 3, such a sliding motion of the ejector 32 leads to a respective lifting and displacement of the cartridge 18 into such a position in which the proximal end 28 of the cartridge 18 sufficiently protrudes from the proximal end 30 of the cartridge holder 11. In this configuration the cartridge 18 can be safely seized and gripped in order to remove and to withdraw it from the cartridge holder.

In this configuration the radially outwardly extending handle portion 42 axially abuts with the proximal end 44 of the longitudinal through opening 26. Even in the event, that the cartridge 18 should be frictionally engaged with the sleeve-shaped ejector body 34, the ejector body 34 is effectively hindered to move beyond the proximal stop position 4 as illustrated in Fig. 3 when the cartridge 18 is withdrawn from the cartridge holder 11. A frictional engagement of ejector body 34 and cartridge is further of advantage when the cartridge holder 11 disconnected from the proximal housing 12 should be turned upside down. Then, an uncontrolled slipping out of the cartridge from the cartridge holder 11 could be effectively prevented.

### List of reference numbers

- 1: distal direction
- 2: proximal direction
- 3: distal stop position
- 4: proximal stop position
- 10: drug delivery device
- 11: cartridge holder
- 12: proximal housing
- 13: distal end
- 14: cartridge holder body
- 15: protective cap
- 16: inspection window
- 17: proximal end
- 18: cartridge
- 19: neck portion
- 20: drive mechanism
- 22: piston rod
- 24: threaded socket
- 25: piston
- 26: through opening
- 28: proximal end
- 30: proximal end
- 31: distal end
- 32: ejector
- 33: distal end
- 34: ejector body
- 35: sidewall
- 36: abutment
- 38: through opening
- 40: slider
- 42: handle portion
- 44: proximal end
- 46: distal end

## Claims

1. A cartridge holder of a drug delivery device, comprising:
- a cartridge holder body (14) extending in a longitudinal axial direction (1, 2) to accommodate a cartridge (18) filled with a medicament,
- an ejector (32) displaceably arranged inside the cartridge holder body (14) between a distal stop position (3) and a proximal stop position (4) and being operably engageable with the cartridge (18) to displace the cartridge (18) in proximal direction (2) relative to the cartridge holder body (14).

2. The cartridge holder according to claim 1, wherein the ejector (32) comprises an ejector body (34) slidably arranged inside the cartridge holder body (14) in axial direction (1, 2).

3. The cartridge holder according to any one of the preceding claims, wherein the ejector (34) comprises an abutment (36) to axially abut with a neck portion (19) of the cartridge (18).

4. The cartridge holder according to claim 3, wherein the abutment (36) comprises a radially inwardly extending flange portion at a distal end (33) of the ejector (32).

5. The cartridge holder according to any one of the preceding claims, wherein the ejector (34) comprises a through opening (38) to receive a radially narrowing neck portion (19) of the cartridge (18).

6. The cartridge holder according to any one of the preceding claims, wherein the ejector (34) comprises a slider (40) seizable from outside the cartridge holder body (14).

7. The cartridge holder according to claim 6, wherein the slider (40) comprises a radially outwardly extending handle portion (42) located in or extending through a longitudinally extending through opening (26) or longitudinally extending slit of a sidewall (35) of the cartridge holder body (14).

8. The cartridge holder according to claim 7, wherein the ejector's (32) proximally directed displacement is delimited by means of the slider (40) or its handle portion (42) axially abutting with a proximal end (44) of the through opening (26) or slit of the cartridge holder body (14)

9. The cartridge holder according to claims 6 to 8, wherein the slider (40) is integrally formed with the ejector body (34).

10. The cartridge holder according to any one of the preceding claims 6 to 9, wherein the slider (40) is located proximally offset from a distal end of the ejector (32).

11. The cartridge holder according to any one of the preceding claims, further comprising a cartridge (18) filled with a medicament and being assembled inside the cartridge holder body (14).

12. The cartridge holder according to claim 11, wherein a proximal end (28) of the cartridge (18) proximally protrudes from a proximal end (30) of the cartridge holder when the ejector (32) is in its proximal stop position (4).

13. A drug delivery device comprising:
- a proximal housing (12) to accommodate a drive mechanism (20) being operably engageable with a piston (25) of a cartridge (18) filled a medicament, and
- a cartridge holder (11) according to any one of the preceding claims to accommodate the cartridge (18) and having a proximal end (30) to releasably connect with a distal end (13) of the proximal housing (12).
